(19) Europäisches Patentamt
European Patent Office
Office européen des brevets

(11) EP 2 520 276 A1

(12) EUROPEAN PATENT APPLICATION

(43) Date of publication:
**07.11.2012 Bulletin 2012/45**

(21) Application number: **11305523.0**

(22) Date of filing: **03.05.2011**

(51) Int Cl.:
***A61K 8/73*** (2006.01) ***A61Q 5/02*** (2006.01)
***A61Q 5/00*** (2006.01) ***C08B 37/00*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Rhodia Opérations**
**93306 Aubervilliers (FR)**

(72) Inventors:
- **Bendejacq, Denis**
  **75005, Paris (FR)**
- **Labeau, Marie-Pierre**
  **Burlington, NJ 08106 (US)**
- **Lizarraga, Gilda**
  **Chesterfield, NJ 08515 (US)**

(74) Representative: **Blot, Philippe Robert Emile**
**Cabinet Lavoix**
**2, place d'Estienne d'Orves**
**75441 Paris Cedex 09 (FR)**

# (54) Use of modified galactomannans for protecting dyed hair color from fading

(57) This invention relates to the use of modified galactomannans in a hair composition for protecting dyed hair color from fading and to a method for protecting dyed hair color from fading by treating said hair with a hair composition containing a modified galactomannan.

EP 2 520 276 A1

## Description

This invention relates to the use of modified galactomannans in a hair composition for protecting dyed hair color from fading. The invention further relates to a method for protecting dyed hair color from fading by treating said hair with a hair composition containing a modified galactomannan.

The coloring of hair has become increasingly popular in recent years. However, fading of artificial hair color has become a common problem and a frequent complaint by consumers. Fading can occur during the shampoo washing treatment as color wash-out, or can be initiated by environmental circumstances, such as by exposure to UV radiation. The washing process is the most significant factor in the removal of hair color, while UV exposure has a significant impact only after 90 hours of intense irradiation (S. Marchloretto, "The Use of Silicones as a Color Lock Aid in Rinse-Off Hair Conditioners", J. of Cosmetic Science, 2003 Annual Scientific Meeting, pp. 130-131). Furthermore, the surfactants present in shampoo formulations provide a wetting function which brings moisture into the hair shaft, thus facilitating the removal of the dye molecules during the water rinsing process.

Maintaining hair color and minimizing hair color fading is highly desirable in the hair care market. Several anti-fading products exist in the market including anti-fading shampoos and conditioners. Some products contain silicones such as dimethicone and amodimethicone, which are believed to effect color retention (see A. Schlosser, "Silicones Used in Permanent and Serai-Permanent Hair Dyes to Reduce the Fading and Color Change Process of Dyed Hair Occurring by Wash Out or UV Radiation", J. Cosmetic Sci, 55 (Supplement), pp.123- 131,2004).

However, there is a continuing need for further improvement.

The aim of the present invention is to provide a hair composition for protecting dyed hair color from fading.

In one embodiment, the present invention is directed to the use of at least one modified galactomannan, in a hair composition, for protecting dyed hair color from fading, wherein said modified galactomannan:

i) has a mean average molecular weight (Mw) from about 50,000 g/mol to about 5,000,000 g/mol; and
ii) contains at least one cationic group, with a cationic degree of substitution ($DS_{cationic}$) from about 0.03 to 0.5.

According to the present invention, the hair composition may contain a mixture of modified galactomannans.

According to the invention, the galactomannan may contain one or several cationic groups, said cationic groups being identical or different.

In the present invention, hair compositions include, but are not limited to, hair conditioner, spray, shampoos, styling gel, serums, masks. Preferably, the hair composition is a shampoo.

The modified galactomannans may be formulated into hair compositions as either leave-on or rinse-off compositions or a combination thereof.

As used herein, the expression "leave-on compositions" designates compositions which are not rinsed with water once applied to the hair.

As used herein, the expression "rinse-off compositions" designates compositions which are rinsed with water once applied to the hair.

As used herein, the expression "dyed hair" means hair which has been colored with a permanent, semi-permanent or temporary artificial color, which can be different from the original color of the hair.

As used herein, the expression "dyed hair color fading" means the color erosion of dyed hair.

Galactomannans are polysaccharides composed principally of galactose and mannose units, wherein the mannose units are linked in a 1-4-β-glycosidic linkage and the galactose branching takes place by means of a 1-6 α-linkage to mannose units. Each ring of the galactose or mannose units (or sugar units) bears three free hydroxyl groups that are available for chemical reaction. The galactomannans are usually found in the endosperm of leguminous seeds such as guar, locust bean, honey locust, flame tree, and the like. In one embodiment, the preferred galactomannan starting material used in the present invention is guar gum, also known as guar.

The average molecular weight of the modified galactomannan suitable for use herein is comprised from about 50,000 g/mol to about 5,000,000 g/mol, preferably from about 200,000 g/mol to about 3,000,000 g/mol, and more preferably from about 500,000 g/mol to about 2,000,000 g/mol. As used herein, the "average molecular weight" of the modified galactomannan means the weight average molecular mass of said galactomannan.

The average molecular weight of the modified galactomannan may be measured by GPC with Light Scattering Detection A value of 0.140 for dn/dc is used for the molecular weight calculations. A Wyatt MALS detector is calibrated using a 22.5 K Da polyethylene glycol standard. All calculations of the molecular weight distributions are performed using Wyatt's ASTRA software. The samples are prepared as 0.05% solutions in the mobile phase (100 mM $Na_2SO_4$, 100 mM $H_3PO_4$) and filtered trough 0.45 μm PVDF filters before analysis.

According to an embodiment of the invention, the modified galactomannan further contains at least one hydrophobic group.

According to the invention, at least one of said hydrophobic group(s) may be borne by a cationic group of the

modified galactomannan or may not be borne by a cationic group of the modified galactomannan.
As used herein, the expression "hydrophobic group may be borne by a cationic group" means that a cationic group bears a hydrophobic group, the hydrophobic group being preferably covalently bound to the cationic group. The hydrophobic group may be directly bound to the cationic group or may be bound with a linker.

According to the present invention, at least one hydrophobic group (g1) is borne by a cationic group of the modified galactomannan and at least one hydrophobic group (g2) is not borne by a cationic group of the modified galactomannan. The modified galactomannan may contain the two types of cationic groups: a cationic group bearing a hydrophobic group and a cationic group which does not bear any hydrophobic group.

In one embodiment, the modified galactomannan may contain at least one type of the following cationic groups: a cationic group which does not bear a hydrophobic group or a cationic group bearing a hydrophobic group. The modified galactomannan may contain one or several cationic group(s) bearing a hydrophobic group or the modified galactomannan may contain one or several cationic group(s) which do(es) not bear a hydrophobic group, or the modified galactomannan may contain both at least one cationic group which does not bear a hydrophobic group and at least one hydrophobic group which is borne by the cationic group.

In one embodiment, the modified galactomannan may contain hydrophobic groups which are all borne by a cationic group or which are all not borne by a cationic group.

In one embodiment, the cationic groups may bear at least two hydrophobic groups, wherein the said hydrophobic groups may be identical or different.

In one embodiment, at least one of the (g1) groups may be identical to at least one of the (g2) groups, preferably all of the (g1) and (g2) groups are identical. The modified galactomannan may contain at least two identical hydrophobic groups wherein one hydrophobic group is borne by a cationic group and the other one is not borne by a cationic group.

In one other embodiment, at least one of the (g1) groups is different from at least one of the (g2) groups, preferably all of the (g1) and (g2) groups are different. The modified galactomannan may contain at least two different hydrophobic groups wherein one hydrophobic group is borne by a cationic group and the other hydrophobic group is not borne by a cationic group.

In one embodiment, the modified galactomannan may contain a mixture of (g1) and (g2) groups, wherein the (g1) and (g2) groups may be identical or different.
In the present invention, the groups (g1) and (g2) are identical when they have the same structure and/or when they have the same chain length.

As used herein, the term "cationic groups" refers to positively charged groups and to partially charged group. As used herein, the expression "partially charged groups" designates groups which may become positively charged depending of the pH of the formulation. Such groups may also be named "potentially cationic groups".
As used herein, the term "cationic" means at least partially cationic. Thus, the terms "cationizing agents", "cationic groups" and "cationic moieties" include ammoniums (which have a positive charge) but also primary, secondary and tertiary amines and their precursors (which can lead to positively charged compounds).

Preferred modified galactomannans for use in the invention include any galactomannans and galactomannan derivatives, such as hydroxyalkyl guar, that have been modified by chemical means, e.g. quaternization, with one or more derivatizing agents containing reactive groups. The modified galactomannans are obtained by reaction between the hydroxyl groups of the galactomannan (and/or the hydroxyl groups of the hydroxyalkyl galactomannan) and the reactive functional groups of the derivatizing agents. Methods for the preparation of the modified galactomannan are disclosed in - U.S. Pat. Nos. 4,663,159; 5,473,059; 5,387,675; 3,472,840; 4,031,307; 4,959,464 and US 2010/0029929, all of which are incorporated herein by reference.

In one embodiment, the modified galactomannans of the invention result from the reaction of any galactomannans and galactomannan derivatives with a cationizing agent and/or a hydrophobizing agent.

Cationizing agents of the present invention are defined as compounds which, by reaction with the hydroxyl groups of the galactomannan can lead to a modified galactomannan comprising at least one cationic group according to the invention.
Cationizing agents of the present invention are defined as compounds which contain at least one cationic moiety or at least one hydrophobic cationic moiety.
As used herein, the term "hydrophobic cationic moiety" means a cationic moiety which contains a hydrophobic moiety. Cationizing agents comprise agents which can lead to cationic modified guars and agents which can lead to cationic hydrophobically modified guars.

Hydrophobizing agents of the present invention are defined as compounds which, by reaction with the hydroxyl groups of the galactomannan can lead to the modified galactomannan comprising at least one hydrophobic group according to the invention.

According to the invention, the cationic groups bearing a hydrophobic group of the modified guars may derive from cationizing agent comprising at least one hydrophobic cationic moiety or from the further reaction of the cationic groups of a modified guar with a hydrophobizing agent as defined below.

According to the invention, the cationic groups which do not bear a hydrophobic moiety of the modified guars are derived from cationizing agents which contain at least one cationic moiety.

According to the invention, the hydrophobic groups of the modified guars are derived from hydrophobizing agents.

A group of suitable derivatizing reagents typically contain a reactive functional group, such as an epoxy group, a halide group, an ester group, an anhydride group or an ethylenically unsaturated group, and at least one cationic moiety or a precursor of such cationic moiety.

As used herein, the term "derivatizing agent" means an agent containing both reactive groups and at least a cationic moiety and/or a hydrophobic moiety which are grafted to a galactomannan.

As used herein, the term "derivatizing agent" also designates a grafting agent.

In one embodiment of the invention, the cationic moieties may be linked to the reactive functional group of the derivatizing agent by a bivalent linking group, such as an alkylene or oxyalkylene group. Suitable cationic moieties include primary, secondary, or tertiary amino groups or quaternary ammonium, sulfonium, or phosphinium groups.

The derivatizing agent can comprise a cationic moiety, or a precursor of a cationic moiety, that contains a cationic nitrogen moiety, more typically, a quaternary ammonium moiety. Typical quaternary ammonium moieties are trialkylammonium moieties, such as trimethylammonium moieties, triethylammonium moieties, or tributylammonium moieties, aryldialkylammonium moieties, such as benzyldimethylammonium moieties, and ammonium moieties in which the nitrogen atom is a member of a ring structure, such as pyridinium moieties and imidazoline moieties, each in combination with a counterion, typically a chloride, bromide, or iodide counterion.

According to the invention, examples of cationizing agents, which lead to cationic modified guars are :

- cationic epoxides, such as 2,3-epoxypropyltrimethylammonium chloride, 2,3-epoxypropyltrimethylammonium bromide ;
- chlorohydrin-functional cationic nitrogen compounds, such as 3-halogeno-2-hydroxypropyl trimethylammonium chloride, for example 3-chloro-2-hydroxypropyl trimethylammonium chloride,
- cationic ethylenically unsaturated monomers or their precursors, such as trimethylammoniumpropyl methacrylamide chloride salt, trimethylammoniumpropyl methacrylamide methylsulfate salt, diallyl dimethyl ammonium chloride, vinyl benzyl trimethylammonium chloride, dimethylaminopropyl methacrylamide (tertiary amine) precursors of cationic monomers, such as N-vinyl formamide, N-vinylacetamide (whose units can be hydrolyzed after polymerization or grafting into vinyl amine units),

According to the invention, examples of cationizing agents, which lead to cationic hydrophobically modified guars are :

- chlorohydrin-functional cationic nitrogen compounds, such as 3-halogeno-2-hydroxypropyl alkyldimethylammonium chloride, for example 3-chloro-2-hydroxypropyl-lauryldimethylammonium chloride, 3-bromo-2-hydroxypropyl-lauryldimethylammonium chloride, 3-chloro-2-hydroxypropyl-stearyldimethylammonium chloride, 3-chloro-2-hydroxypropyl-cocoalkyldimethylammonium chloride or 3-bromo-2-hydroxypropyl-stearyldimethylammonium chloride;
- cationic ethylenically unsaturated monomers or their precursors, such as benzyl-dimethylammonium ethyl methacrylate chloride or salts of alkyl-dimethyl ammonium propyl methacrylamide; (meth)acrylamide-functional nitrogen compounds such as terbutyl amino ethyl methacrylate (secondary amine);

According to one embodiment of the invention, examples of hydrophobizing agents, which lead to hydrophobically modified guars are :

- epoxides, such as glycidyl ethers of aliphatic alcohols wherein the aliphatic alcohol contain about 5 to about 25 carbon atoms, such as glycidyl ethers of amyl alcohol, hexanol, octanol, lauryl alcohol, stearyl alcohol, lignoceryl alcohol; or such as hexadecyl glycidylether;
- 1-2-epoxy alkanes, such as 1,2-epoxyoctane, 1,2-epoxydodecane, 1,2-epoxyhexadecane, 1,2-epoxytetracosane and epoxidized alpha-olefins from Arkema (Vikolox®);
- 1-2-epoxypolybutenes, such as epoxidized polybutenes from Arkema (Vikopol®);
- halides, such as alkyl halides, in particular octyl chlorides, dodecyl iodide, decyl bromide, hexadecyl bromide and the like;
- fatty acids and fatty esters, such as saturated fatty acids comprising between 8 and 12 carbon atoms or such as methyl octanoate, methyl decanoate, methyl dodecanoate, methyl tetradecanoate, methyl hexadecanoate, methyl octadecanoate, or vegetal oils such as copra or palm-kernel oil;
- anhydrides of fatty acids, such as alkenyl succinic anhydride ester, alkenyl succinic acid ester, acetic anhydride, octanoic anhydride;

- benzylic derivatives, such as benzyl chlorides, benzyl esters, benzyl anhydrides, benzyl epoxides.

Preferably, the hydrophobizing agents are chosen from :

- epoxides, such as glycidyl ethers of aliphatic alcohols wherein the aliphatic alcohol contain about 5 to about 25 carbon atoms, such as glycidyl ethers of amyl alcohol, hexanol, octanol, lauryl alcohol, stearyl alcohol, lignoceryl alcohol; triethylene glycol monoglycidyl ether, triethylene glycol diglycidyl ether; or such as hexadecyl glycidylether;
- 1-2-epoxy alkanes, such as 1,2-epoxyoctane, 1,2-epoxydodecane, 1,2-epoxyhexadecane, 1,2-epoxytetracosane and epoxidized alpha-olefins from Arkema (Vikolox®);
- 1-2-epoxypolybutenes, such as epoxidized polybutenes from Arkema (Vikopol®);
- halides, such as alkyl halides, in particular octyl chlorides, dodecyl iodide, decyl bromide, hexadecyl bromide and the like;
- fatty acids and fatty esters, such as saturated fatty acids comprising between 8 and 12 carbon atoms or such as methyl octanoate, methyl decanoate, methyl dodecanoate, methyl tetradecanoate, methyl hexadecanoate, methyl octadecanoate, or vegetal oils such as copra or palm-kernel oil;
- anhydrides of fatty acids, such as alkenyl succinic anhydride ester, alkenyl succinic acid ester, acetic anhydride, octanoic anhydride;

More generally speaking, the derivatization of guars can be done with agents such as epoxides, halides, aldehydes, ethylenically unsaturated monomers, carboxylic acids, esters or their anhydrides, such as alkenyl succinic anhydride ester, alkenyl succinic acid ester or caprylic acid.

In the case of a derivatizing agent containing ethylenically unsaturated function(s), the grafting can be achieved at basic pH (for example by Michael addition of the monomer onto the hydroxyl groups of the galactomannan) or by radical polymerization according to methods known as "grafting from" or "grafting onto" for example.

In a preferred embodiment, the hydrophobic groups may be incorporated into a modified galactomannan possessing tertiary amine moiety (precursor of a cationic moiety) by reaction with an alkyl chloride.

Preferably, the hydrophobic groups of the modified galactomannan are chosen from linear or branched alkyl groups containing from 8 to 22 carbon atoms, preferably from 11 to 19 carbon atoms, and more preferably from 12 to 18 carbon atoms.

According to the invention, the cationic groups may be introduced into a galactomannan by reacting the galactomannan starting material with a derivatizing agent which comprises a reactive functional group and at least one cationic moiety (or a precursor of cationic moiety).

According to the invention, the cationic groups present in the modified galactomannan are incorporated into the galactomannan starting material by reaction of the hydroxyl groups of said galactomannan with a cationizing agent. Preferred cationic groups are chosen from the group consisting of : primary, secondary or tertiary amino groups, quaternary ammonium, sulfonium or phosphinium groups, and mixtures thereof. In a particular preferred embodiment, the cationic groups are trialkylammonium groups, such as trimethylammonium groups, triethylammonium groups, tributylammonium groups, aryldialkylammonium groups, such as benzyldimethylammonium groups, and ammonium groups in which the nitrogen atom is a member of a ring structure, such as pyridinium groups and imidazoline groups, each in combination with a counterion, typically a chloride, bromide, or iodide counterion. Preferably, each cationic group contains at least one cationic charge.

According to the present invention, the modified galactomannan has a cationic degree of substitution ($DS_{cationic}$) from about about 0.03 to 0.5, preferably from about 0.065 to 0.35, and more preferably from about 0.08 to 0.30.
As used herein, the expression "cationic degree of substitution" ($DS_{cationic}$) means the average number of moles of cationic groups per mole of sugar unit. The $DS_{cationic}$ may be measured by means of $^1$H-NMR (solvent: $D_2O$ or DMSO).

In another preferred embodiment, the cationic hydrophobically modified galactomannan have a hydrophobic degree of substitution ($DS_{hydrobobic}$) from about 0.0012 to 0.5, preferably from about 0.0015 to 0.2, and more preferably from about 0.0015 to 0.1
As used herein, the term "hydrophobic degree of substitution" ($DS_{hydrophobic}$) means the average hydrophobic substitution on the hydroxyl groups of the galactomannan per mole of sugar unit. The ($DS_{hydrobobic}$) may be measured by means of $^1$H-NMR (solvent : $D_2O$ or DMSO).

The term "modified galactomannan" in the present invention encompasses the terms modified galactomannan polymer, cationic modified galactomannan and cationic hydrophobically modified galactomannan.
As used herein, the expression "cationic modified galactomannan" designates modified galactomannan comprising at least one cationic group.
As used herein, the term "cationic hydrophobically modified galactomannan" designates modified galactomannan comprising at least one cationic group and at least one hydrophobic group, wherein the hydrophobic group may be borne or not by the cationic group of the modified galactomannan.

In the present invention, the preferred galactomannan is a guar.

In one preferred embodiment, the modified galactomannan comprises at least one unit having the general formula (I):

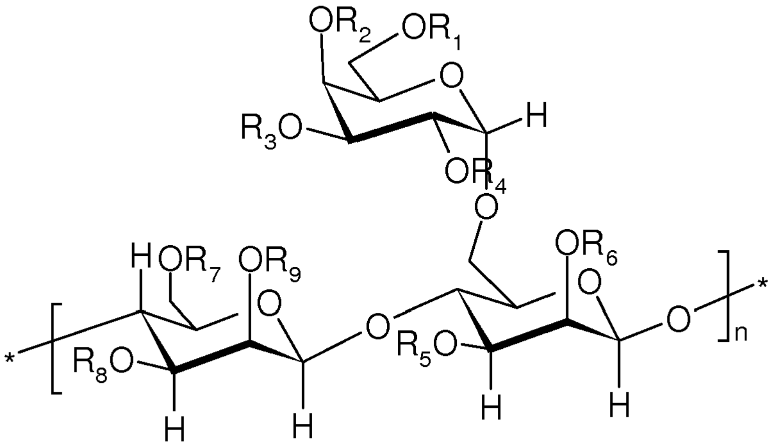

(I)

wherein n is comprised between 20 to about 50,000, each of R1, R2, R3, R4, R5, R6, R7, R8 and R9 represents H, a cationic group or a hydrophobic group, and at least one of R1, R2, R3, R4, R5, R6, R7, R8 and R9 is a cationic group.

In a preferred embodiment, the modified galactomannan comprises at least one unit having the general formula (I):

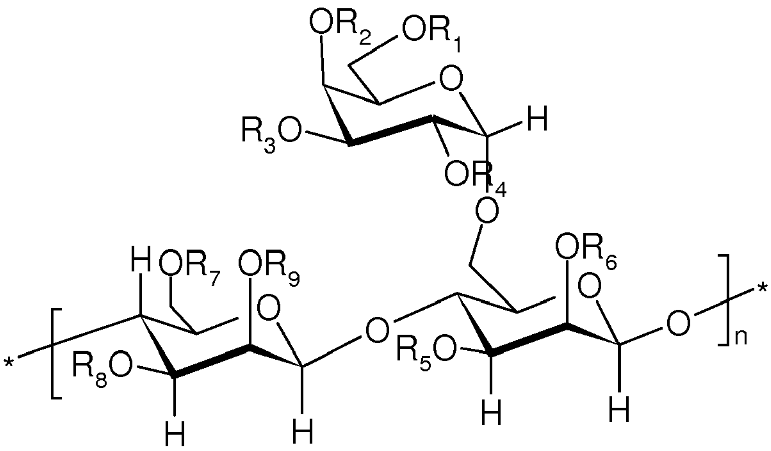

wherein each of R1, R2, R3, R4, R5, R6, R7, R8 and R9 represents H or a cationic group bearing a hydrophobic group, and at least one of R1, R2, R3, R4, R5, R6, R7, R8 and R9 is a cationic group bearing a hydrophobic group.

In one preferred embodiment, the modified galactomannan comprises at least one unit having the general formula (I):

12

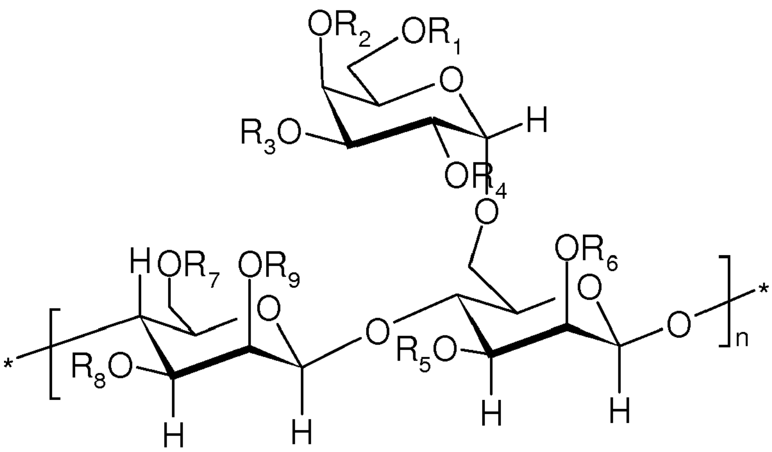

(I)

wherein :

- each of R1, R2, R3, R4, R5, R6, R7, R8 and R9 represents H or a cationic group having the formula (II) :

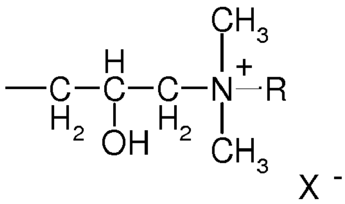

(II)

wherein n is from 20 to about 50,000, X represents an halogen atom and R represents a linear or branched alkyl group containing from 8 to 22 carbon atoms ;
- and at least one of R1, R2, R3, R4, R5, R6, R7, R8 and R9 is a cationic group having the formula (II).

The group R of formula (II) is preferably selected from the group consisting of cocoalkyl, C12 and C18, and X is preferably Cl.

Preferably, the cationic groups of formula (II) have one of the following formulae:

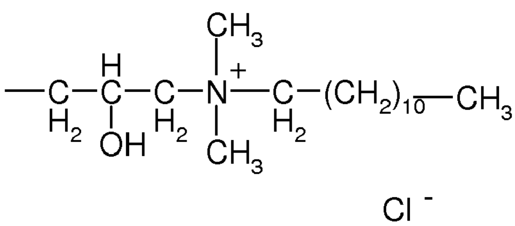

or

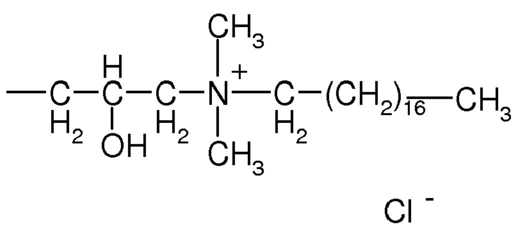

or

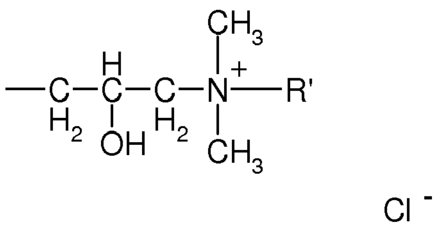

with R' = cocoalkyl.

The present invention also relates to a method for protecting dyed hair color from fading, which comprises treating said dyed hair with a hair composition, comprising a modified galactomannan which:

i) has a mean average molecular weight (Mw) from about 50,000 g/mol to about 5,000,000 g/mol; and
ii) comprises at least one cationic group, with a cationic degree of substitution ($DS_{cationic}$) from about 0.03 to 0.5.

This modified galactomannan is as defined above.
According to a preferred embodiment, the cationic groups of the modified galactomannan used in the method of the invention also contain hydrophobic groups. Preferably, the hydrophobic groups of the modified galactomannan are chosen from linear or branched alkyl groups containing from 8 to 22 carbon atoms, preferably from 11 to 19 carbon atoms, and more preferably from 12 to 18 carbon atoms.
Preferably, the cationic groups of the modified galactomannan as used in the method of the invention have one of the following formulae :

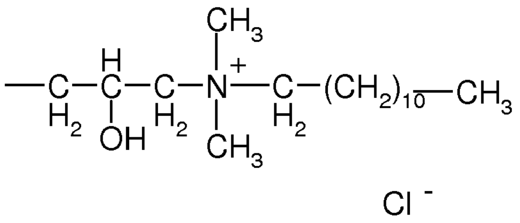

or

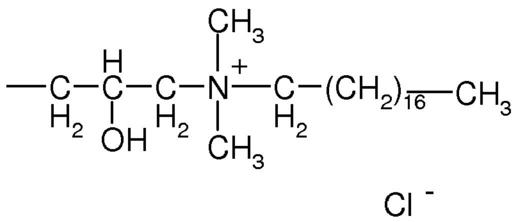

or

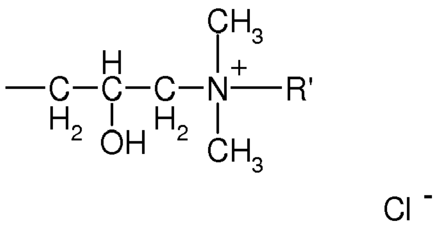

with R' = cocoalkyl.
According to the invention, the method for protecting dyed hair color from fading, consists in contacting the dyed hair with a hair composition, comprising the modified galactomannan, and then, if appropriate, rinsing said hair with water.
It has been unexpectedly found, that the use of a modified galactomannan when incorporated into a functional hair composition is effective in protecting dyed hair color from fading. This discovery forms the basis of the current invention.
In a preferred embodiment, the dyed hair used in the present invention is double-bleached, mono-bleached and/or permed hair.
The expression "mono-bleached hair" used herein means hair which has been discolored once, with an oxidizing agent.
The expression "double-bleached hair" used herein refers to hair which has been discolored with an oxidizing agent, twice.
The following examples will serve to illustrate the invention, all parts and percentages being by weight, unless otherwise indicated.

## Examples

A. Hair tress preparation

• Tress pre-treatment

Hair tresses were supplied by International Hair Importers and Products Inc, Lots: 2-gram Caucasian medium brown tresses, damaged in different ways (bleached once, twice). Before the dyeing process, the tresses were pre-treated with a detergent solution: each tress was put under a controlled water flow (1.6 L/min at 37°C) for 1 minute, then massaged for 1 min with 1 mL of a 10wt% SLES (INCI Sodium laureth sulphate) solution and rinsed for 1 minute.

• Tress dying

A commercial coloration kit was rapidly applied on the tresses in the most homogeneous way possible. After 45 min, the coloration was stopped on all tresses at the same time in a large water bath; then, each tress was rinsed for 30 seconds (6.0 L/min at 37°C) and then 60 seconds (1.6 L/min at 37°C).
Finally, the tresses were dried for 2 hours in a 40°C-oven. Initial color coordinates $L_O a_O b_O$ were determined 10 times along each colored tress with a Konika-Minolta spectrocolorimeter 2500d. Among the tresses colored, only those closest in terms of color coordinates were kept for the study.

B. Shampooing methodologies

Any shampoo was applied only after 48 hours. The shampooing process consists in the following: each tress was put under a controlled water flow for 1 minute (1.6 L/min at 37°C), then pressed between two fingers to get rid of the excess water; a dose of shampoo of 0.4 grams was applied on the 2-gram tress (i.e. 20wt% shampoo / hair) and the tress massaged for 45 seconds. Each tress was rinsed under a water flow for 45 seconds, then combed and pressed between two fingers to get rid of the extra water. All tresses were placed in an oven at 40 °C for 2 hours. This step is repeated as many times as required, up to 10 shampoo cycles.
Each shampoo considered was tested on 3 to 5 tresses for reproducibility's sake. A batch of 20 colored tresses therefore allowed 4 to 6 different shampoos to be tested.

C. Measurement methods

After the Nth shampoo/drying cycle, color coordinates $L_N a_N b_N$ were determined 10 times along each colored tress with a Konika-Minolta spectrocolorimeter 2500d. Color fading ΔE(N) of each was then computed according to:

$$\Delta E(N) = \sqrt{(L_N - L_O)^2 + (a_N - a_O)^2 + (b_N - b_O)^2}$$

N being the number of shampoos already performed. The associated changes in L and and b coordinates:

$$\Delta L(N) = L_N - L_O \quad \Delta a(N) = a_N - a_O \quad \Delta b(N) = b_N - b_O$$

were also computed. These different quantities were averaged over the 3 to 5 tresses on which the same shampoo was tested.
The measurement of ΔE(N) highlights the change in color. The larger value of ΔE(N) reflects greater change of color, hence, color fading.

D. Synthesis of the polymers

Meaning of abbreviations or acronyms used in the synthesis examples:

QUAB® 342: 3-chloro-2-hydroxypropyl-lauryl-dimethylammonium chloride
QUAB® 360 : 3-chloro-2-hydroxypropyl-cocoalkyl-dimethylammonium chloride
QUAB® 426: 3-chloro-2-hydroxypropyl-stearyl-dimethylammonium chloride
QUAT® 188 : 3-chloro-2-hydroxypropyl- trimethylammonium chloride
SLES : Sodium Lauryl Ether Sulfate
CAPB : Cocamidopropyl Betaine

Suppliers:

QUAB® 360, QUAB® 342, QUAB® 426: Degussa Chemical Company
Quat®188 : Quab Specialties
Mirataine :Rhodia
Rhodapex : Rhodia

Kathon : Rohm and Haas

After each synthesis, the final product is analyzed by SEC-MALS (size exclusion chromatography with detection by multi-angle light-scattering detection). The average molar masses are expressed by weight. The degree of cationic substitution ($DS_{cationic}$) and the degree of hydrophobic substitution ($DS_{hydrophobic}$) were analyzed by 1 H NMR (solvent : $D_2O$ or DMSO) and express the average number of moles of cationic and hydrophobic substitution, respectively, per mole of sugar unit.

The average molecular weights of the modified galactomannan were measured by GPC with Light Scattering using the following conditions :

Column : Shodex OH pak SB-806M HQ, 3 columns
Mobile phase :100 mM $Na_2SO_4$, 100 Mm $H_3PO_4$
Flow rate : 1.0 ml/min
Detector : Agilent Refractive Index Detector, Wyatt miniDAWN MALS detector
Inj. volume : 200 μl
Temperature : ambient
Run time : 50 minutes

A value of 0.140 for dn/dc was used for the molecular weight measurements. The Wyatt MALS detector was calibrated using a 22.5 K Da polyethylene glycol standard. All calculations of the molecular weight distributions were performed using Wyatt's ASTRA software. The samples were prepared as 0.05% solutions in the mobile phase and filtered trough 0.45 μm PVDF filters before analysis.

Modified galactomannans of use in the present invention were made in the following manner :

Example 1 : synthesis of Polymer 1

The modified galactomannan polymer 1 was made according to patent EP 0 686 643. The molecular weight was Mw= 2-3 x$10^6$ g/mol and the $DS_{cationic}$= 0.18-0.28.

Example 2: synthesis of Polymer 2

The modified galactomannan polymer 2 was made according to patent EP 0 686 643. The molecular weight was Mw= 300-650 x$10^3$ g/mol and the $DS_{cationic}$= 0.09-0.14.

Example 3 : synthesis of Polymer 3

The modified galactomannan polymer 3 was made according to patent US 5,756, 720. The molecular weight was Mw= 1-1.5 x$10^6$ g/mol and the $DS_{cationic}$= 0.10-0.13.

Example 4 : synthesis of Polymer 4

The modified galactomannan polymer 4 was made using the following reagents in the ensuing amounts and using methods known to those skilled in the art, such as those published on US 5,756,720.
Guar (Rhodia, Mw = 2 x$10^6$ g/mol) : 100g (0.49 moles)
2-propanol: 181g (3.01 moles)
De-ionized water: 32g
QUAB 342, 39% (Mw = 342 g/mol): 43g (0.05 moles)
Sodium hydroxide, 25%: 9g (0.06 moles)
The molecular weight for the product of the synthesis in Example 4 was Mw=2.0 x$10^6$ g/mol and the $DS_{hydrophobic}$=0.027. $DS_{cationic}$= 0.27.

Example 5 : synthesis of Polymer 5

The modified galactomannan polymer 5 was made using the following reagents in the ensuing amounts and using methods known to those skilled in the art, such as those published on US 5,756,720.
Guar (Rhodia, Mw = 2 x$10^6$ g/mol) :100g (0.49 moles)
2-propanol: 181g (3.01 moles)
De-ionized water: 32g

QUAB® 426, 39% (Mw = 426 g/mol): 160g (0.15 moles)
Sodium hydroxide, 25%: 9g (0.06 moles)
The molecular weight for the product of the synthesis in Example 5 was Mw=2,61 x$10^6$ g/mol and the $DS_{hydrophobic}$=0.024. $DS_{cationic}$= 0.27.

Example 6: synthesis of Polymer 6

The modified galactomannan polymer 6 was made using the following reagents in the ensuing amounts and using methods known to those skilled in the art, such as those published on EP 0 686 643.
Guar splits (Rhodia, Mw = 2-3x$10^6$ g/mol): 500g
Water: 369g
Sodium hydroxide: 50%, 184g
Hydrogen peroxide: 20%, 35g
Sodium tetraborate: 3%, 98g
Quat® 188, 69% (Mw = 188 g/mol): 389g
The molecular weight measured for the product of the synthesis in Example 6 was Mw=2.1x$10^5$ g/mol and the $DS_{cationic}$=0.19.

Example 7: synthesis of polymer 7

The modified galactomannan polymer 7 was made using the following reagents in the ensuing amounts and using methods known to those skilled in the art, such as those published on EP 0 686 643.
Guar splits (Rhodia, Mw = 2-3x$10^6$ g/mol): 500g
Water: 244 g
Sodium hydroxide: 50%, 81 g
Hydrogen peroxide: 20%, 28 g
Sodium tetraborate: 3%, 48 g
Quat® 188, 69% (Mw = 188 g/mol): 174 g
The molecular weight measured for the product of the synthesis in Example 7 was Mw=4.1x$10^5$ g/mol and the $DS_{cationic}$=0.108.

Example 8: synthesis of polymer 8

The modified galactomannan polymer 8 was made using the following reagents in the ensuing amounts and using methods known to those skilled in the art, such as those published on US 5,756,720.
Guar (Rhodia, Mw = 2 x$10^6$ g/mol): 150g (0.735 moles)
2-propanol: 196g, (3.25 moles)
De-ionized water: 75g
QUAB® 360, 38.7% (Mw = 360 g/mol): 41 g, (0.044 moles)
Sodium hydroxide, 30%: 31 g, (0.2 moles)
The molecular weight for the product was Mw=2-3 x$10^6$ g/mol with $DS_{hydrophobic}$=0.010 and $DS_{cationic}$=0.27.

E. Preparation of the formulation comprising the polymers

Several shampoos were prepared the following way in 200-mL vessels equipped with a mechanical stirring:

- under moderate stirring, the dry powdered galactomannan polymer was slowly added in distilled water; once well dispersed, the galactomannan polymer was hydrated by adjusting the pH to 3.5 - 5 with citric acid; the clarity of the solution should improve. Then, the preparation was stirred an additional 15 to 20 minutes with moderate stirring to ensure proper hydration;
- raw material Mirataine BET C30 (cocamidopropyl betaine) was added and the mixture was stirred until fully homogeneous;
- raw material Rhodapex ES 2K (sodium lauryl ether sulfate) was added and the solution was stirred until fully homogeneous;
- NaCl (sodium chloride) was next added and the mixture was stirred until fully homogeneous;
- Kathon CG (preservative) was added;
- the pH was adjusted to 5.5 - 6.

Table 1 indicates the different formulations created with the different polymers. Formulations C to J are based on the 8 polymers of examples 1 to 8, respectively. Two formulations serve as references :

- formulation A consists of a commercial benchmark (Elsève® Color Vive) renowned in color protection and available in stores in Europe, based on a guar hydroxypropyltrimonium chloride according to its INCI list, a polymer of the same family as those described in examples 5 to 8.
- formulation B consists of a surfactant base with 14wt% SLES, 2wt% CAPB and 1.6wt% NaCl.

Table 1 : Formulations prepared with the different polymers.

| FORMULATIONS | A | B | C | D | E | F | G | H | I | J |
|---|---|---|---|---|---|---|---|---|---|---|
| Benchmark | 100 | 0 | 0 | 0 | 0 | 0 | 0 | 0 | 0 | 0 |
| Rhodapex ES 2K (SLES, 25.7wt% active material) | 0 | 54.5 | 54.50 | 54.50 | 54.50 | 54.50 | 54.50 | 54.50 | 54.50 | 54.50 |
| Mirataine BET C30 (CAPB, 29.8wt% active material) | 0 | 6.70 | 6.70 | 6.70 | 6.70 | 6.70 | 6.70 | 6.70 | 6.70 | 6.70 |
| NaCl | 0 | 1.60 | 1.60 | 1.60 | 1.60 | 1.60 | 1.60 | 1.60 | 1.60 | 1.60 |
| Kathon CG | 0 | 0.05 | 0.05 | 0.05 | 0.05 | 0.05 | 0.05 | 0.05 | 0.05 | 0.05 |
| Citric acid | 0 | pH3.5-5 | pH3.5-5 | pH3.5-5 | pH3.5-5 | pH3.5-5 | pH3.5-5 | pH3.5-5 | pH3.5-5 | pH3.5-5 |
| water | 0 | 37.15 | 36.85 | 36.85 | 36.65 | 36.65 | 36.65 | 36.65 | 36.65 | 36.65 |
| Polymer 1 (Example 1) | 0 | 0 | 0.3 | 0 | 0 | 0 | 0 | 0 | 0 | 0 |
| Polymer 2 (Example 2) | 0 | 0 | 0 | 0.3 | 0 | 0 | 0 | 0 | 0 | 0 |
| Polymer3 (Example 3) | 0 | 0 | 0 | 0 | 0.5 | 0 | 0 | 0 | 0 | 0 |
| Polymer 4 (Example 4) | 0 | 0 | 0 | 0 | 0 | 0.5 | 0 | 0 | 0 | 0 |
| Polymer 5 (Example 5) | 0 | 0 | 0 | 0 | 0 | 0 | 0.5 | 0 | 0 | 0 |
| Polymer 6 (Example 6) | 0 | 0 | 0 | 0 | 0 | 0 | 0 | 0.5 | 0 | 0 |
| Polymer 7 (Example 7) | 0 | 0 | 0 | 0 | 0 | 0 | 0 | 0 | 0.5 | 0 |
| Polymer 8 (Example 8) | 0 | 0 | 0 | 0 | 0 | 0 | 0 | 0 | 0 | 0.5 |

F. Performances of the modified galactomannans on mono-bleached hair

The performances of formulations containing modified galactomannans with different molecular weights and different $DS_{cationic}$ were evaluated on mono-bleached medium brown Caucasian hair. The formulations A, C and D are based on the same type of polymer Guar hydroxypropyltrimonium chloride.

Table 2 indicates the results of color fading from dyed hair samples after 5 shampoos.

Table 2 : ΔE(N) after 5 shampoos

| Formulations | Polymers | ΔE(N) |
|---|---|---|
| A | Standard commercial guar | $2.71^{\pm 0\ 21}$ |
| C | Polymer 1 | $2.38^{\pm 0\ 19}$ |
| D | Polymer 2 | $2.03^{\pm 0\ 27}$ |

The results in Table 2 show that formulations C and D are more effective, after 5 shampoos, in reducing hair dye fading than the formulation A comprising the commercial benchmark (Elsève® Color Vive). The formulation D containing modified galactomannan polymer 2 is the most effective in reducing color erosion of mono-bleached hair.

G : Performances of hydrophobically modified galactomannans on mono-bleached hair

The performances of formulations B, C and J were evaluated on mono-bleached medium brown Caucasian hair. The formulation J is based on the same type of polymer (guar hydroxypropyltrimonium chloride) with the formulation C (polymer 1), wherein the said polymer is further hydrophobized.

Table 3 provides the results of color fading from dyed hair samples after 10 shampoos.

Table 3: ΔE(N) after 10 shampoos

| Formulations | Polymers | Mw (g/mol) | $DS_{cationic}$ | $DS_{hydrophobic}$ | ΔE(N) |
|---|---|---|---|---|---|
| C | Polymer 1 | $2\text{-}3x10^6$ | 0.18-0.28 | 0 | $10.12^{\pm 0.62}$ |
| B | None | - | - | - | $11.17^{\pm 0.19}$ |
| J | Polymer 8 | $2\text{-}3x10^6$ | 0.27 | 0.010 | $9.09^{\pm 0.36}$ |

The results in Table 3 show that the formulation J (polymer 8) leads to the smallest value of 4E(N) compared to formulations C (polymer 1) and B (surfactant base). Advantageously, the introduction of hydrophobic groups in a cationic polymer leads to the reduction of the hair dye fading of mono-bleached hair.

H : Performances of modified galactomannans on double-bleached hair

The performances of formulations E, H and I were evaluated on double-bleached medium brown Caucasian hair.

Table 4 provides the results of color fading from dyed hair samples after 10 shampoos.

Table 4 : After 10 shampoos

| Formulations | Polymers | Mw (kg/mol) | $DS_{cationic}$ | ΔE(N) |
|---|---|---|---|---|
| E | Polymer 3 | 1000-1500 | 0.10-0.13 | $8.40^{\pm 0.40}$ |
| H | Polymer 6 | 210 | 0.19 | $7.64^{\pm 0.13}$ |
| I | Polymer 7 | 410 | 0.108 | $9.25^{\pm 0.17}$ |

The results in Table 4 show that the formulation H (polymer 6) leads to the smallest value of ΔE(N) compared to formulations E (polymer 3) and I (polymer 7). The formulation H comprises a galactomannan polymer with a smaller molecular weight and a higher $DS_{cationic}$ than the polymer of formulation E. Advantageously, the use of a modified galactomannan having a small MW and a high $DS_{cationic}$ leads to the reduction of the hair dye fading of double-bleached hair.

I : Performances of hydrophobically modified galactomannans on double-bleached hair

The performances of formulations A, F and G were evaluated on double-bleached medium brown Caucasian hair.

Table 5 provides the results of color fading from dyed hair samples after 10 shampoos.

Table 5 : After 10 shampoos

| Formulations | Polymers | Alkyl chain length | $DS_{hydrophobic}$ | ΔE(N) |
|---|---|---|---|---|
| A | Standard commercial guar | - | 0 | $9.02^{\pm 0 12}$ |
| F | Polymer 4 | C12 | 0.027 | $7.59^{\pm 0 30}$ |
| G | Polymer 5 | C18 | 0.024 | $7.76^{\pm 0 45}$ |

The results in Table 5 show that the formulations F and G lead significantly to smaller values of ΔE(N) than formulation A (Elsève® Color Vive) which contains a standard commercial guar. Hence, the introduction of hydrophobic groups into cationic galactomannan polymer allows reducing color fading of double-bleached dyed hair. Advantageously, the use of a hydrophobically modified galactomannan polymer leads to the reduction of the hair dye fading of double-bleached hair.

## Claims

**1.** Use of at least one modified galactomannan, in a hair composition, for protecting dyed hair color from fading, wherein said modified galactomannan:

i) has a mean average molecular weight (Mw) from about 50,000 g/mol to about 5,000,000 g/mol; and
ii) contains at least one cationic group, with a cationic degree of substitution ($DS_{cationic}$) from about 0.03 to about 0.5.

**2.** Use according to claim 1, wherein the modified galactomannan further contains at least one hydrophobic group.

**3.** Use according to claim 2, wherein (at least one of) said hydrophobic group(s) is(are) borne by a cationic group of the modified galactomannan.

**4.** Use according to claim 2, wherein (at least one of) said hydrophobic group(s) is(are) not borne by a cationic group of the modified galactomannan.

**5.** Use according to claim 4, wherein the hydrophobic groups are derived from hydrophobizing agent chosen from: epoxides, halides, fatty acids, fatty esters, and anhydrides of fatty acids.

**6.** Use according to any one of claims 1 to 5, wherein at least one hydrophobic group (g1) is borne by a cationic group of the modified galactomannan and at least one hydrophobic group (g2) is not borne by a cationic group of the modified galactomannan.

**7.** Use according to claim 6, wherein at least one of the (g1) groups is identical to at least one of the (g2) groups, preferably all of the (g1) and (g2) groups are identical.

**8.** Use according to claim 6, wherein at least one of the (g1) groups is different from least one of the (g2) groups, preferably all of the (g1) and (g2) groups are different.

**9.** Use according to any one of claims 1 to 8, wherein the hydrophobic group of the modified galactomannan is chosen from linear or branched alkyl groups containing from 8 to 22 carbon atoms.

**10.** Use according to any one of claims 1 to 9, wherein the hydrophobically modified guar has a hydrophobic degree of substitution ($DS_{hydrobobic}$) from about 0.0012 to 0.5.

**11.** Use according to any one of claims 1 to 10, wherein the modified galactomannan has a molecular weight from about 200,000 g/mol to about 3,000,000 g/mol.

**12.** Use according to any one of claims 1 to 11, wherein the modified galactomannan has a molecular weight from about 500,000 g/mol to about 2,000,000 g/mol.

**13.** Use according to any one of claims 1 to 12, wherein the cationic groups are chosen from the group consisting of: primary, secondary or tertiary amino groups, quaternary ammonium, sulfonium or phosphinium groups, and mixtures thereof.

**14.** Use according to any one of claims 1 to 13, wherein the cationic groups are chosen from : trialkylammonium groups, such as trimethylammonium groups, triethylammonium groups, or tributylammonium groups, aryldialkylammonium groups, such as benzyldimethylammonium groups, and ammonium groups in which the nitrogen atom is a member of a ring structure, such as pyridinium groups and imidazoline groups.

**15.** Use according to any one of claims 1 to 14, wherein the galactomannan is a guar.

**16.** Use according to any one of claims 1 to 15, wherein the modified galactomannan comprises at least one unit having the general formula (I):

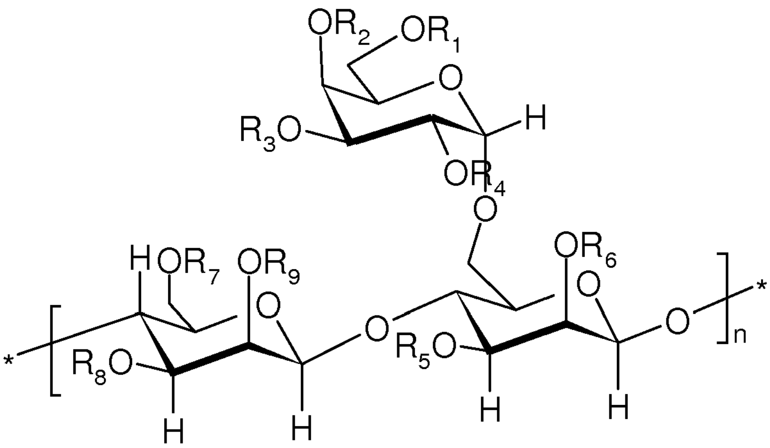

(I)

wherein n is comprised between 20 to about 50,000; each of R1, R2, R3, R4, R5, R6, R7, R8 and R9 represents H, a cationic group or a hydrophobic group, and at least one of R1, R2, R3, R4, R5, R6, R7,R8 and R9 is a cationic group.

**17.** Use according to claim 16, wherein each of R1, R2, R3, R4, R5, R6, R7, R8 and R9 represents H or a cationic group bearing a hydrophobic group.

**18.** Use according to any one of claims 1 to 17, wherein the modified galactomannan comprises at least one unit having the general formula (I):

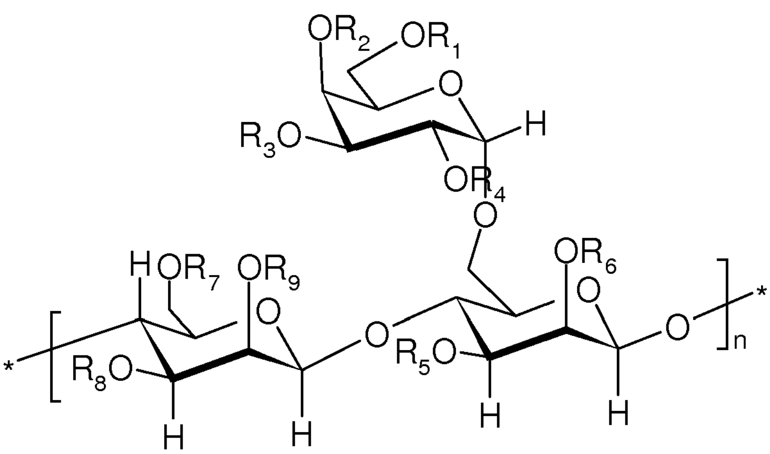

(I)

wherein :

- each of R1, R2, R3, R4, R5, R6, R7, R8 and R9 represents H or a cationic group having the formula (II) :

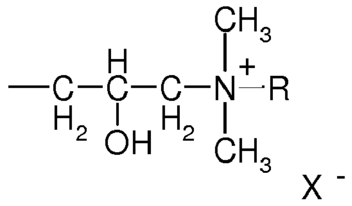

(II)

wherein n is from 20 to about 50,000, X represents an halogen atom and R represents a linear or branched alkyl group containing from 8 to 22 carbon atoms ; and at least one of R1, R2, R3, R4, R5, R6, R7 , R8 and R9 is a cationic group having the formula (II).

**19.** Use according to claim 18, wherein the cationic groups of formula (II) have one of the following formulae:

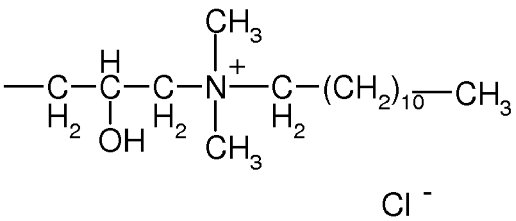

or

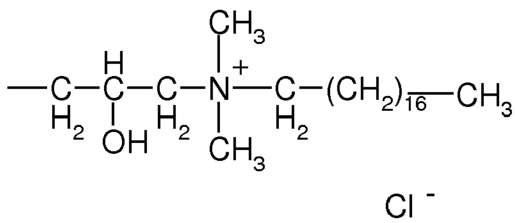

or

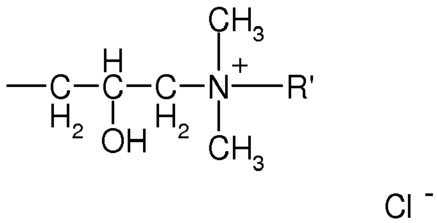

with R' = cocoalkyl.

**20.** A method for protecting dyed hair color from fading, which comprises treating said dyed hair with a hair composition comprising at least one modified galactomannan which:

i) has a mean average molecular weight (Mw) from about 50,000 g/mol to about 5,000,000 g/mol; and
ii) comprises at least one cationic group, with a cationic degree of substitution ($DS_{cationic}$) from about 0.03 to about 0.5.

**21.** The method of claim 20, wherein the dyed hair is double-bleached, mono-bleached or permed hair.

Europäisches Patentamt
European Patent Office
Office européen des brevets

# EUROPEAN SEARCH REPORT

Application Number
EP 11 30 5523

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/091557 A2 (HERCULES INC [US]) 28 October 2004 (2004-10-28)<br>* page 1, line 18 - line 22 *<br>* page 4, line 6 - line 17 *<br>* page 7, line 6 - line 15 *<br>* examples *<br>----- | 1-21 | INV.<br>A61K8/73<br>A61Q5/02<br>A61Q5/00<br>C08B37/00 |
| Y | FR 2 944 967 A1 (OREAL [FR]) 5 November 2010 (2010-11-05)<br>* page 1, line 34 - page 2, line 8 *<br>* page 4, line 13 - page 5, line 16 *<br>* page 17, line 19 - line 26 *<br>* page 17, line 40 - page 18, line 2 *<br>* example 1 *<br>----- | 1-21 | |
| Y,D | US 5 756 720 A (CHOWDHARY MANJIT SINGH [US]) 26 May 1998 (1998-05-26)<br>* column 2, line 60 - column 3, line 20 *<br>* examples *<br>----- | 1-21 | |

TECHNICAL FIELDS SEARCHED (IPC)

A61K
A61Q
C08B

The present search report has been drawn up for all claims

1

| Place of search | Date of completion of the search | Examiner |
|---|---|---|
| Munich | 25 October 2011 | Ruckebusch, Virginie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 11 30 5523

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2011

| Patent document cited in search report | Publication date | Patent family member(s) | Publication date |
|---|---|---|---|
| WO 2004091557 A2 | 28-10-2004 | BR PI0409243 A | 28-03-2006 |
| | | CA 2519373 A1 | 28-10-2004 |
| | | CN 1780857 A | 31-05-2006 |
| | | EP 1611157 A2 | 04-01-2006 |
| | | JP 2006522829 A | 05-10-2006 |
| | | KR 20050120790 A | 23-12-2005 |
| | | MX PA05010749 A | 15-12-2005 |
| FR 2944967 A1 | 05-11-2010 | NONE | |
| US 5756720 A | 26-05-1998 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4663159 A
- US 5473059 A
- US 5387675 A
- US 3472840 A
- US 4031307 A
- US 4959464 A
- US 20100029929 A
- EP 0686643 A
- US 5756720 A


### Non-patent literature cited in the description


- **S. MARCHLORETTO.** The Use of Silicones as a Color Lock Aid in Rinse-Off Hair Conditioners. *J. of Cosmetic Science, 2003 Annual Scientific Meeting,* 2003, 130-131
- **A. SCHLOSSER.** Silicones Used in Permanent and Serai-Permanent Hair Dyes to Reduce the Fading and Color Change Process of Dyed Hair Occurring by Wash Out or UV Radiation. *J. Cosmetic Sci,* 2004, vol. 55, 123-131